# EUROPEAN PATENT SPECIFICATION

(11) **EP 4 422 560 B1**
(45) Date of publication and mention of the grant of the patent: **02.07.2025**
(21) Application number: 22801545.9
(22) Date of filing: 20.10.2022
(51) Int. Cl.: A61F 2/58

(54) **ANATOMICAL PROSTHESIS WITH A MECHANISM FOR INDEPENDENT FINGER ACTUATION**
ANATOMISCHE PROTHESE MIT EINEM MECHANISMUS ZUR UNABHÄNGIGEN FINGERBETÄTIGUNG
PROTHÈSE ANATOMIQUE AVEC MÉCANISME D'ACTIONNEMENT INDÉPENDANT DU DOIGT

(30) Priority: 29.10.2021 IT 202100027836
(43) Date of publication of application: 04.09.2024
(73) Proprietor: Fondazione Istituto Italiano di Tecnologia, 16163 Genova (IT); I.N.A.I.L. Istituto Nazionale per l'Assicurazione Contro gli Infortuni sul Lavoro, 00144 Roma (IT)
(72) Inventor: BOCCARDO, Nicolò, 16159 GENOVA (IT); STEDMAN, Samuel, 16145 GENOVA (IT); ROSSI, Paolo, 16165 GENOVA (IT); LAFFRANCHI, Matteo, 16128 GENOVA (IT); DE MICHIELI, Lorenzo, 16148 GENOVA (IT); CANEPA, Michele, 16128 GENOVA (IT); GRUPPIONI, Emanuele, 40013 CASTEL MAGGIORE (BO) (IT)
(74) Representative: Metroconsult Srl
(86) International application number: PCT/IB2022/060083
(87) International publication number: WO 2023/073512

(56) References cited:
- WO-A1-2019/215577
- GB-A- 2 537 898
- GB-A- 2 591 213
- US-A1- 2018 098 862

## Description

The present invention relates, in general, to anatomical limb prostheses, and particularly, but not exclusively, to prostheses for upper limbs, i.e. hands.

Before proceeding any further, it is worth pointing out that, although in the following description and in the appended claims reference will mostly be made to hand prostheses, the information provided may be extended also to other types of prostheses and/or robotic devices controllable by means of cable-type differential transmission mechanisms.

As is known, for assisting people who have suffered serious hand (or foot) amputations, anatomical prostheses have been quite recently developed which can reproduce not only the shape of the amputated limb, but also some of its functions.

Such functions include, for example, grasping objects or exerting pulling or pressing forces and performing other actions that may seem simple and obvious to normal people, but that, in order to be implemented in an artificial limb, require a very complex structure and operation. In fact, such prostheses have a large number of miniaturized mechanical components that require a complex control system for reproducing movements similar to those of a normal limb. In some cases, such movements are controlled by the person by means of gestures or muscular contractions that require an interface between the prosthesis and the muscular or nervous system, as well as suitable sensors (e.g. electrodes, optical sensors, etc.) positioned on the person's body.

Developing an artificial arm having functions equivalent to those of the human arm is a general goal of bioengineering.

In the current state of the art, prostheses still lack some degrees of freedom and compel individuals to compensate for them by means of compensating movements, which often cause pain to the residual limb and overuse syndromes.

The prosthetic or myoelectric hand is applied to the arm, or the remaining part thereof, in order to enhance or assist its capabilities, e.g. in terms of force and/or speed of movement.

For this purpose, the prosthesis is secured at predefined locations, particularly at the arm joint (the elbow), and is actuated by passive devices (e.g. springs, elastic cords, etc.) and/or by active devices (e.g. electric or pneumatic actuators, etc.) which, also by means of mechanical transmission systems (which may include, for example, gears, pulleys, cables, belts, etc.), actuate the movement of part of the prosthetic hand, e.g. some fingers or the wrist, and the transfer of forces and torques between the parts, as is the case of opposable fingers, and between them and the body.

In the prosthetic limbs known in the art, the actuation systems apply forces to the various limb segments in a way as compliant as possible with the anatomical movements, exploiting the properties of elastic or viscoelastic components.

This is aimed at transmitting a force to the prosthesis and its parts which is as gradual and anatomically compliant with the real limb as possible, in order to avoid any sudden acceleration or hard contact with the actuation system; this is somewhat similar to a requirement that can be found in the automotive field, wherein the drive torque generated by the engine must be transmitted gradually and seamlessly to the wheels to avoid stresses and malfunctions.

The present invention belongs to the category of active prosthetic devices, the main function of which is to assist the user's physical effort when carrying out activities such as lifting and handling objects.

As far as the prosthetic or myoelectric hands considered herein are concerned, they essentially include an anchorage part or base to be anchored to the amputated arm, a main body connected in an articulated manner to the base by means of a joint or wrist that can be rotated relative to the anchorage base, and one or more fingers hinged to the body.

Each finger is made up of a series of mutually hinged phalanges.

Prosthetic hands may also include one electric actuator for each one of the degrees of freedom of the hand, and an actuator control unit.

The use of one actuator for each degree of freedom of the hand makes the latter inevitably complex, heavy and bulky; this is why underactuated hands have been invented in the past, i.e. hands having a number of actuators smaller than the number of degrees of freedom of the hand. One example of such a hand is described in international patent application WO 2019/215577 (corresponding to Italian patent IT 102018000005213) granted to the same Applicants of the present application.

The prosthesis disclosed in the above document includes a system for independently actuating the hand's fingers, i.e. an actuation system acting upon some fingers separately from others.

In particular, as explained in the above-mentioned prior-art document, the known prosthetic hand comprises a base body that can be rotated relative to an articulation of the anchorage wrist, and one or more fingers hinged to the main body; each finger is made up of a series of mutually hinged phalanges.

The base body identifies the palm of the underactuated prosthetic hand, to which the fingers are hinged in pairs, i.e. a first index finger-middle finger pair and a second ring finger-little finger pair.

In addition, the myoelectric hand is provided with a supplementary finger acting as a thumb, which is adapted to work in opposition to the other fingers for grasping objects.

The phalanges of each finger are mutually articulated, and within them a flexible cable extends which, running from the tip to the base, allows flexing and extending the finger, such cable being tensioned or slackened by means of a system of pulleys and idlers controlled by an actuator.

In brief, according to the solution described in document WO 2019/215577, the actuation system includes a primary cable that is wound and unwound by the actuator against the elastic action of return springs.

The length variations occurring when winding or unwinding the primary cable result in tensioning or slackening of the secondary cables that cause flexion or extension of the fingers connected thereto.

To this end, the actuation mechanism comprises sliders or slides that are movable along guides extending in the hand's longitudinal direction, whereon idler pulleys are arranged for the primary cable and for each one of the secondary cables to be moved.

This known myoelectric hand has the advantage that by means of a single actuator it is possible to control the movements of two pairs of front fingers (index finger-middle finger and ring finger-little finger) and of the opposable lateral finger (thumb), such a hand being thus underactuated, i.e. requiring fewer actuators than the number of degrees of freedom actually controlled. However, prosthetic devices exist which can move each finger independently by means of a dedicated motor.

Unlike underactuated ones, such hands and their actuation systems may also permit a three-finger grasp, wherein the three-finger grasp is achieved by means of suitable gear mechanisms and cannot be modified, so that the functionality of the prosthetic limb is limited to a single grasp type.

In light of such encouraging results, the present Applicants have felt the need for improving the performance of the electromyographic prosthetic hand of WO 2019/215577.

In particular, the attention has been focused on the fact that it would be appropriate to enhance the functionality of the prosthetic limb so as to have the possibility of actuating the fingers of the prosthesis independently, i.e. not all fingers together as in the known solution.

To the Applicants' knowledge, in the current state of the art this function is obtained through the use of specific electromechanical actuators, at least one of which is dedicated to controlling the opposable finger or thumb finger.

One example of this state of the art is found in patent publication US 2013/046395.

It follows that such a solution is not compatible with an underactuated prosthesis like the one addressed by the present invention, i.e. prostheses having fewer actuators than degrees of freedom.

In other words, it can be stated that the technical problem tackled by the invention is to provide an underactuated limb prosthesis, in particular, without being limited thereto, a hand prosthesis, which can independently actuate at least three fingers.

The fingers may be either single, like the thumb, or paired, e.g. index finger-middle finger and ring finger-little finger.

With this problem in mind, the invention aims at creating a prosthesis that is light and that can be advantageously be integrated into an existing prosthetic structure without requiring many modifications and without requiring significant energy consumption additional to the energy consumption necessary for actuating the prosthesis.

The idea that solves this technical problem is to provide the prosthesis with means for independently locking or stopping the movements of the fingers, so that, when the user commands the actuation of some of them, the other ones will remain in a flexed or extended or intermediate position, according to the case.

Preferably, the stopping means are movable between a first operative condition, in which they stop the movements of some fingers and/or their phalanges, and a second non-operative condition, in which they allow them to move freely.

The features of the invention are more specifically set out in the claims appended to this description.

Such features will become clearer in the light of the following description of an exemplary embodiment of the invention, wherein reference will be made to the annexed drawings, provided merely by way of non-limiting example, wherein:
- Fig. 1 is a perspective view of a hand according to the invention, in the open condition;
- Fig. 2 shows the hand of Fig. 1 closed into a fist;
- Figs. 3 and 4 show respective longitudinal sectional views of the preceding hand in the conditions of Figs. 1 and 2 and of the respective configurations of the underactuation mechanism;
- Fig. 5 is a side view, with an area partially sectioned and magnified, of the hand of the preceding figures, which shows part of the underactuation mechanism;
- Fig. 6 is a longitudinal sectional view of a finger of the hand of the preceding figures;

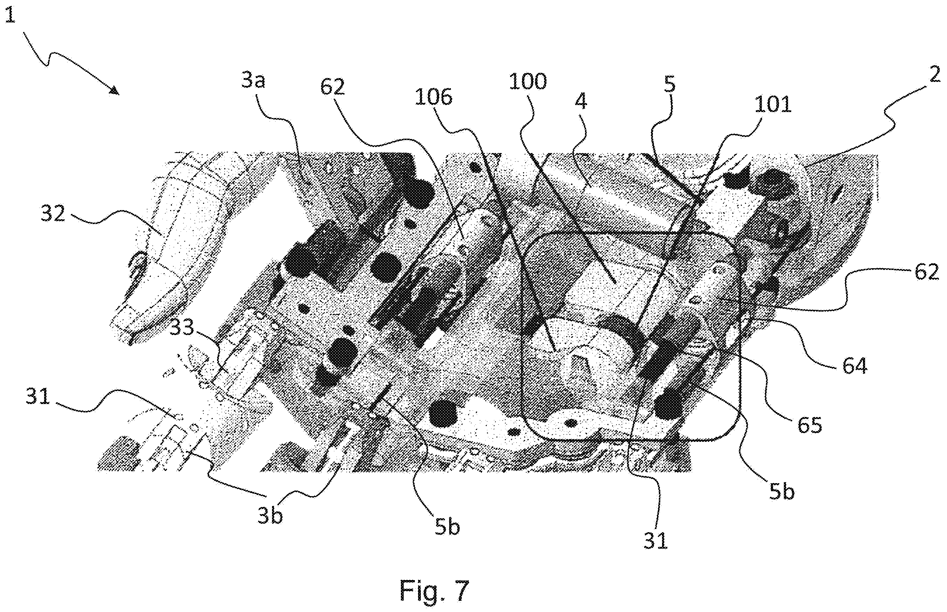

- Fig. 7 shows an inside view of a portion of the hand of Figures 1-6;
- Figs. 8 and 9 show a perspective view and a longitudinal view, respectively, of a device for stopping the fingers of the hand according to the invention, visible in Fig. 7;
- Fig. 10 is a diagram showing the position and operation of the stopping device of Figs. 8 and 9;
- Figs. 11(a)-11(f) show respective operating conditions of the hand according to the invention. With reference to the above-listed drawings, 1 designates as a whole a hand in accordance with the invention.

For simplicity and clarity, reference will be made herein to an electromyographic prosthetic hand like the one described in WO 2019/215577, suitably modified as will be further explained below.

This should not, however, be understood as limiting, conflicting with or departing from the present invention, nor any conclusions may be drawn by extrapolating parts of the description from the context and the teaching of the present disclosure.

In this frame, it must be pointed out that any reference to "an embodiment" in the present description will indicate that a particular configuration, structure or feature is comprised in at least one possible embodiment of the invention.

Therefore, expressions like "in an embodiment" and the like, which may be found in different parts of this description, will not necessarily refer to the same embodiment. Moreover, any particular configuration, structure or feature may be combined as deemed appropriate in one or more embodiments, even if not expressly shown or described herein, in accordance with the teaching of the invention or the knowledge of those skilled in the art.

The references below, many of which correspond to those found in WO 2019/215577, are used only for simplicity's sake, and shall not limit the protection scope or extension of the invention and of the various embodiments thereof.

With this in mind, it can be stated that the prosthetic hand 1 comprises a base body 2 and a plurality of fingers 3 hinged or connected in an articulated manner to the base body 2; the fingers 3 preferably correspond to those of a real hand, i.e. index finger, middle finger, ring finger and little finger.

The base body 2 is adapted to be constrained to an external element, such as a robotic or human forearm, not shown in the drawings; for this purpose, the base body 2 may be connected to the robotic forearm in an articulated, or anyway rotatable, manner to make it similar to a wrist, or may be rigidly connected to the forearm.

Both options are applicable to the hand 1 of the invention.

The base body 2 defines a prevalent portion of the prosthesis, substantially corresponding to a portion of the back 2a and/or a portion of the palm 2b of the prosthetic hand 1, from which the fingers 3 extend.

The latter preferably comprise at least two fingers 3b, 3c hinged to the base body 2 and separately, or anyway selectively, controllable; in particular, the hand 1 preferably comprises two pairs of prosthetic fingers 3b, 3c, which correspond, respectively, to the index finger-middle finger 3b and ring finger-little finger 3c pairs.

Each finger pair 3b, 3c preferably comprises one proximal phalanx 31 connected to the base body 2 of the hand and a distal phalanx 32 at the extremity of the corresponding finger, mutually articulated to be flexed and extended according to the operating conditions of the prosthetic hand 1 along corresponding axes or directrices of rotation 31a, 32a.

In addition, according to a preferred embodiment of the invention, the hand 1 comprises also a supplementary finger 3a adapted to work in opposition to one or more other fingers 3b, 3c to facilitate grasping or seizing object. The supplementary finger 3a can be identified as a thumb, as shown in the drawings, and also comprises a proximal phalanx 31 and a distal phalanx 32 mutually articulated to allow flexing the thumb 3a relative to an axis 31a.

The proximal phalanges 31 of the fingers 3a, 3b, 3c are hinged to the base body 2 of the hand 1, thereby defining a first axis of rotation 31a.

In this exemplary embodiment, each prosthetic finger 3a, 3b, 3c comprises a joint 33 to which the respective proximal phalanx 31 is constrained; since the joint 33 is part of, or anyway protrudes from, the base body 2 of the hand, it follows that also the respective proximal phalanx 31 is constrained to the base body 2.

Optionally, the prosthetic hand 1 may comprise a mechanism (not shown in the drawings) for moving one or more fingers 3a, 3b, 3c, which can rotate relative to it, closer to or away from each other or the base body 2.

The prosthetic hand 1 is underactuated in that it has fewer actuators than the number of degrees of freedom of its movable components (i.e. the fingers).

Preferably, it comprises an actuator 4 suitable for controlling the actuation of one or more fingers 3a, 3b, 3c through the use of a system of cables 5. The actuator is typically an electric motor, usually a (brushed or brushless) direct-current motor, possibly associated with a reducer or anyway with means 4a for varying the speed of an output shaft 4b, whereon a primary cable 5a is wound or unwound.

Since the prosthetic hand 1 is actuated by just one actuator 4, it falls under the definition of "single-actuator" hand.

The actuator 4 is adapted to control the flexion and extension of the phalanges 31, 32 of the fingers 3; in particular, the proximal phalanx 31 rotates relative to the base body 2 about the first axis of rotation 31a, whereas the distal phalanx 32 rotates relative to the proximal phalanx 31 about the second axis of rotation 32a. The actuator 4 is located upstream of the fingers 3 along the kinematic chain of the prosthetic hand 1; more specifically, it is constrained to the base body 2, e.g. by shape coupling and/or fastening means such as screws and the like.

The actuator 4 is preferably electric and powered by a battery that may be incorporated into the structure of the prosthetic device or into the anchorage structure for anchoring the device to the patient; the electric battery may be connected by means of a cable, and may even be placed at a distance from the patient.

In order to actuate the phalanges 31, 32 of the fingers 3, the prosthetic hand 1 comprises at least one first cable, or primary control cable, 5a, connected to the actuator 4, which moves it by winding or unwinding it according to the hand's operating phases.

The system for actuating the fingers 3 of the hand 1 further comprises at least one secondary cable, or driven cable, 5b, which extends along one or more finger pairs 3b, 3c, i.e. index finger-middle finger or ring finger-little finger, for controlling the flexion and extension thereof. The primary 5a and secondary 5b cables are operatively connected to each other by a transmission assembly 6, adapted to allow the first control cable 5a to control the second control cable 5b.

The hand 1, which comprises two pairs of prosthetic fingers 3b (index finger-middle finger), 3c (ring finger-little finger), is preferably equipped with a single primary cable 5a and two secondary cables 5b dedicated to a respective one of the finger pairs 3b, 3c.

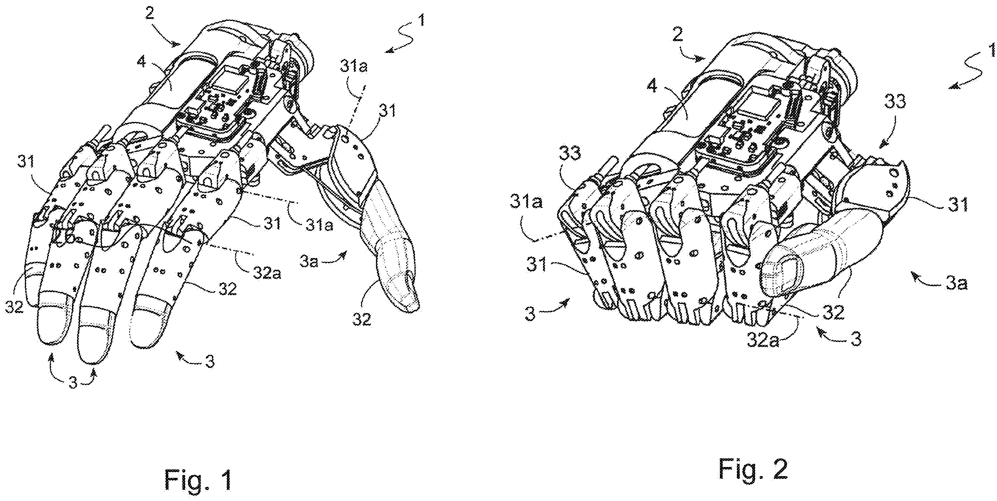

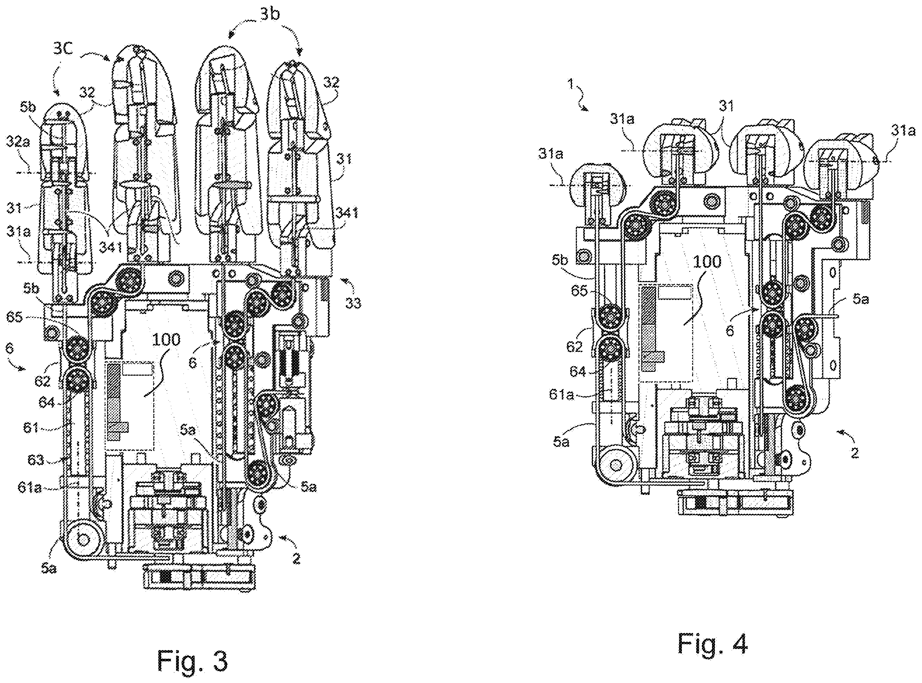

The primary cable 5a and the secondary cables 5b are connected by means of two transmission assemblies 6, so that, following a command imparted by the actuator 4, the primary cable 5a can act simultaneously upon both secondary cables 5b (Figs. 2 and 3).

The cables 5a, 5b are made of light and strong materials, thus being able to withstand the stresses generated in use, even after many operating cycles; preferably, they are made of polyethylene, or fibres derived from such polymer, in that this material combines good mechanical properties with a low friction coefficient, thus facilitating the sliding action of the cables 5a, 5b.

In an embodiment, the primary cable 5a has a first end secured to, or anyway integral with, the base body 2 of the hand, and a second end integral with the actuator 4.

In a prosthetic hand comprising an opposable thumb finger 3a as in the example considered herein, the first cable 5a is adapted to control such thumb finger 3a, and its first control end is integral with the opposable finger 3a. In accordance with a preferred embodiment, the end of the primary cable 5a is connected to the distal phalanx 32 of the opposable finger 3a.

Each one of the two secondary cables 5b has a first control end constrained to a first finger of a respective pair 3b, 3c, i.e. index middle finger finger and finger-ring-little finger, while the second end of the secondary cables 5b is constrained to the second finger of such pairs of prosthetic fingers 3b, 3c.

Preferably, each secondary cable 5b has its first control end respectively associated with the distal phalanx 32 of the first finger 3b (index finger) and 3c (ring finger), while the second control end of the secondary cables 5b is respectively associated with the distal phalanx 32 of the second finger 3b (middle finger) and 3c (little finger).

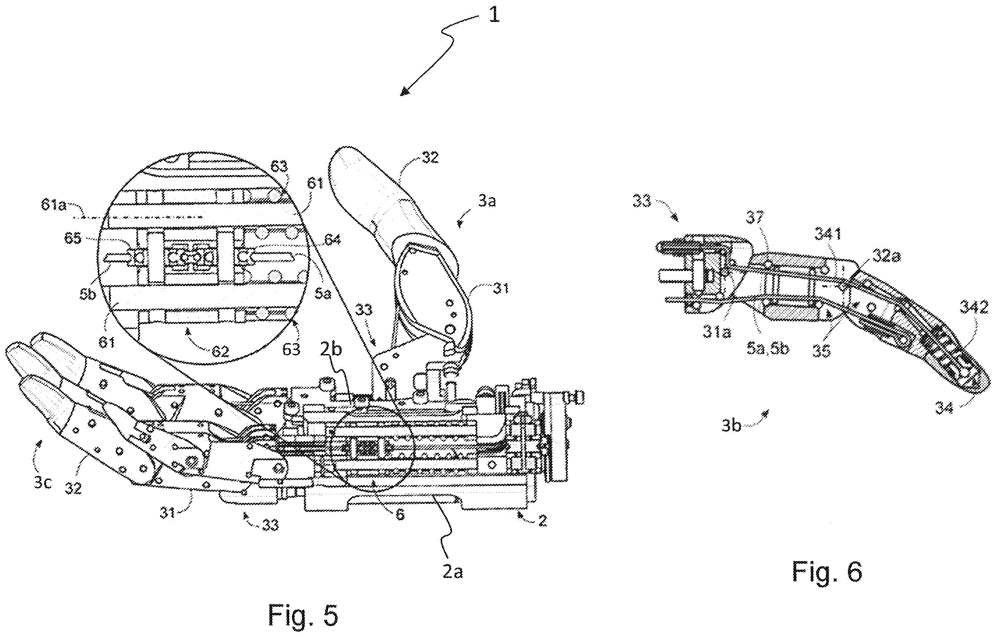

The transmission assemblies 6 (see Fig. 5) comprise each:
at least one guide 61 integral with the base body 2;
a slider or movable element 62 slidable along the guide 61;
elastic means 63, preferably coil springs, although they may consist of different elements (Belleville washers, gas springs or the like), adapted to elastically counter the sliding motion of the slider or movable element 62 along the guide 61;
a first pulley or roller 64, hinged to the movable element 62, whereon the primary cable 5a can slide;
a second pulley or roller 65, hinged to the movable element 62, whereon each secondary cable 5b can slide, so that the actuator 4, when it drives the primary cable 5a, will cause a translation of the movable element 62 along the guide 61.

Preferably, each transmission assembly 6 comprises two parallel guides 61, along which the respective movable element 62 can slide; the guides 61 of the two blocks 6 are substantially parallel to each other.

The translation of the movable element 62 along the guides 61 causes, since the pulleys 64 are hinged thereto, the respective secondary cable 5b to be pulled (tensioned) and/or released (slackened), thereby moving the prosthetic fingers 3 in accordance with the translation direction.

In more detail, the translation of the movable element 62 is transformed into a rotation, preferably a concordant one, of the phalanges 31 and 32 about the axes 31a and 32a in a first direction. It must be pointed out that, at the same time, a rotation of the opposable finger 3a may occur relative to the base body 2 in a first direction, generating the grip with the other fingers 3b, 3c.

In this respect, it must be specified that in this document the "first direction" identifies a rotation controlled by the actuator 4, whereas the "second direction" identifies a rotation opposite the one in the first direction.

The translation of the movable element 62 occurs in opposition to the elastic countering action exerted by the elastic means 63, which, when the actuator 4 is off (i.e. when it is not applying a torque/force to the primary cable 5a), cause the movable element to return to the initial position.

In more detail, the action of the elastic means 63 permits a rotation, conveniently in the same direction, of the phalanges 31 and 32 of the prosthetic fingers 3b, 3c, in a second direction opposite the first direction and bringing said fingers 3b, 3c into the initial position.

At the same time, the return of the movable element 62 into the initial position causes a rotation or extension of the opposable finger 3a in a second direction, opposite the first one.

The guides 61 are linear and define a sliding axis or direction 61a for the respective movable element 62; the two guides 61 have their sliding axes 6a substantially parallel to each other. The pulleys 64 and 65 define, relative to the corresponding movable element 62, axes of rotation substantially parallel to each other and substantially perpendicular to the sliding axis 61a. Alternatively, said axes of rotation may be mutually inclined.

The elastic means 63 are adapted to keep the primary cable 5a under tension; to this end, they are preferably preloaded.

The elastic means 63 preferably comprise at least one coil spring coaxial to the sliding axis 61a; preferably, there is at least one coil spring 63 arranged around the respective guide 61. The coil spring 63 is adapted to work by extension and/or, preferably, also by compression. Each prosthetic finger 3a, 3b, 3c comprises a return device 34 adapted to work in opposition to a rotation or flexion of the finger, particularly of the phalanges 31 and 32, commanded by the actuator 4 through the primary cable 5a and secondary cable 5b.

For this purpose, the return device 34 is adapted to rotate the phalanges 31 and 32 in the second direction, i.e. in the extension direction.

It must be pointed out that this motion is preferably controlled by the return device 34 alone, while the elastic means 63 facilitate such motion by slackening the secondary cable 5b.

The return device 34 comprises a cable 341 having a first end constrained to the base body 2 of the hand or to the joint 33 provided thereon, and a second end constrained to the respective distal phalanx 32; the return device comprises an elastic member 342, such as a spring or a rubber block or the like, adapted to keep the secondary cable 5b under tension.

The return cable 341 has its second end integral with the elastic body 342, which is interposed between said second end and the distal phalanx 32. The return cable 341 preferably lies on the side opposite the primary control cable 5a and/or the secondary control cable 5b, with reference to the axes of rotation 31a and 32a.

Preferably, the return cables 341 of the fingers 3b, 3c of a hand 1 are on the opposite side with respect to all axes of rotations 31a and 32a of the phalanges 31, 32 and the control cables 5a and/or 5b, so that the fingers 3a, 3b, 3c can rotate in the same direction when they are actuated by either the return cables 341 or the control cables 5a and/or 5b.

The elastic body 342 is adapted to keep the return cable 341 under tension and to work in opposition to the control cables 5a and/or 5b.

Therefore, when the actuator 4 is activated, the control cable 5a and/or 5b rotates the phalanges 31 and 32 in the first direction and moves the return cable 341, thus loading the elastic body 342, which, as the actuator 4 is deactivated, can command a rotation of the phalanges 31 and 32 in the second direction.

The elastic body 342 is housed in the distal phalanx 32 and preferably consists of a coil spring coaxial to the distal phalanx 32. In more detail, it may be a spring working by extension and/or compression, depending on the implementations of the hand control mechanism.

The elastic body 342 may be preloaded to adjust the elastic response of the fingers 3a, 3b, 3c. Preferably, the fingers 3a, 3b, 3c have elastic bodies 342 with different preloads, differentiating their response and hence the finger movements.

In accordance with the present invention, the hand's fingers 3b, 3c, i.e. the index finger-middle finger and ring finger-little finger pairs, perform movements that may be mutually independent. To this end, devices 100 for stopping or locking the transmission assemblies 6 are provided in proximity to the guides 61.

The stopping devices 100 comprise a cam mechanism 101 associated with an actuator 102; the latter preferably consists of a brushless direct-current electric motor 103, coupled to a mechanical reducer 104 or the like. In a preferred solution, the reducer is a planetary gear reducer with a 700:1 ratio.

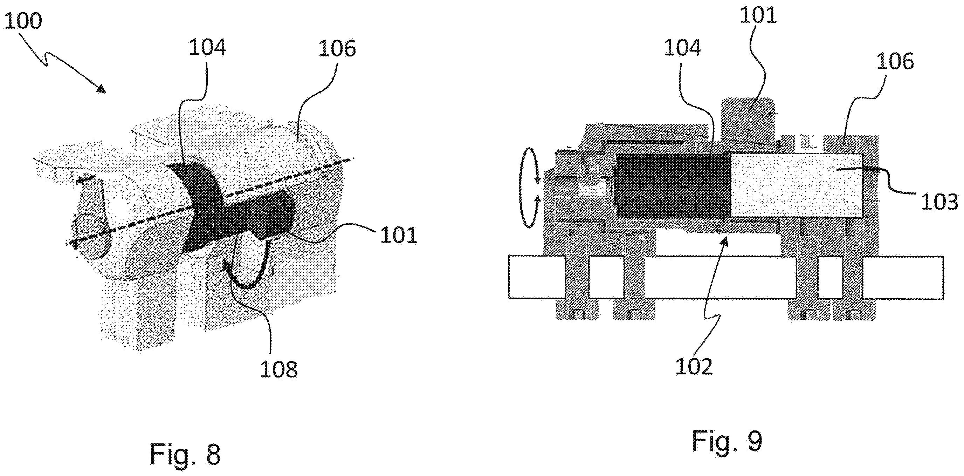

The actuator 102 controls the rotation of a cam 101 that moves between a non-operative condition (visible in Fig. 9), in which it is substantially aligned with the body 106 of the device 100, and an operative condition, in which it is rotated relative to the Z axis, preferably coinciding with the longitudinal axis of the motor 103 and reducer 104.

In the operative condition, the cam 101 protrudes from the body 106 of the device (visible in Fig. 8) and prevents the sliding movements of the slider 62 of a respective transmission assembly 6 along the corresponding guide 61.

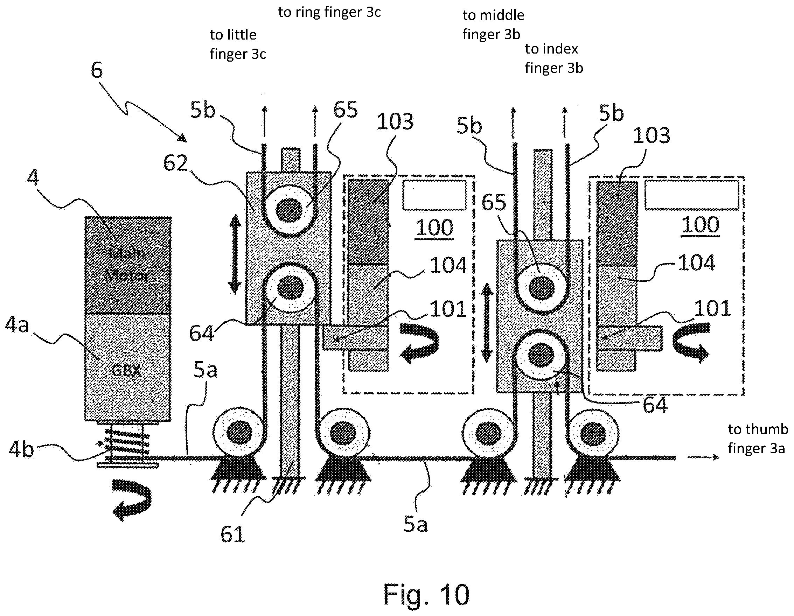

The operating condition is schematically shown in Figure 10 with reference to the transmission assembly on the left in the figure (assembly 100), associated with the ring finger-little finger pair 3c.

Advantageously, in accordance with a preferred embodiment, when the cam 101 is rotated into the non-operative configuration of the mechanism (Fig. 8), it engages into a groove 108 of the body 106 of the device, so as to keep the stopping device 100 in a locked condition, followed by deactivation of the actuator 103.

Vice versa, when the cam 101 is rotated in the opposite direction (away from the groove 108), the operating condition in which the adjacent slider 62 is retained can be maintained without the motor of the actuator 103 using electric energy, resulting in clear advantages when using the prosthetic hand 1.

In its non-operative condition, therefore, the cam 101 is retracted from the outline of the device 100, thus letting the adjacent slider 62 move freely; this situation is visible in the right part of Figure 10, with reference to the index finger-middle finger pair 3b. Vice versa, in the operating condition shown in Figure 10 on the left, the cam 101 intercepts the stroke of the slider 62, thereby limiting the movement of the ring finger-little finger pair (3c) and facilitating the movement of the index finger-middle finger pair (3b) and possibly also of the thumb (3a). From the above description one can understand the operation of the prosthetic hand 1 and of the separate, or anyway selective, mode of actuation of the fingers 3, in particular of the triplet of fingers or finger pairs 3a, 3b, 3c.

Let us start from the working condition in which the system for stopping the triplet of fingers is disabled and the fingers 3a, 3b, 3c are free to move, i.e. they are free to flex or extend for closing or opening the hand.

In this situation, the stopping device 100 is activated by at least one EMG (electromyographic) signal sent by the user (amputee) for grasping an object, or anyway for separately controlling the finger pairs (index finger-middle finger, ring finger-little finger) 3b, 3c and the opposable thumb 3a.

The amputee can control the opening and closing of the prosthetic hand 1 by means of EMG signals, which cause the rotation of the electric motor of the main actuator 4. The activation of this motor 4 in a first direction of rotation causes the primary cable 5a to be wound on the output shaft 4b of the differential mechanism 4a, which is connected to the motor's output. The winding of the primary cable 5a causes a downward movement of the sliders 62 of the two transmission assemblies 6, with their respective pulleys or rollers 64, 65, along their respective linear guides 61.

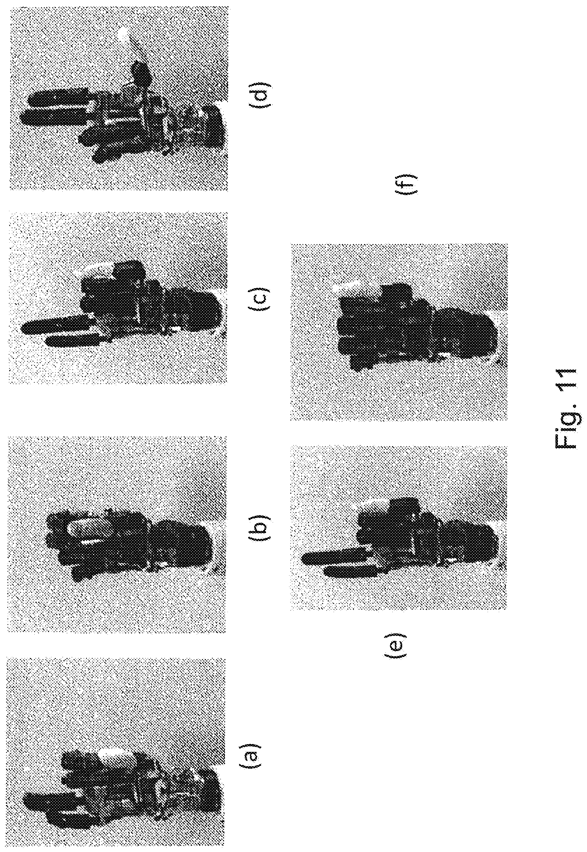

The movement of the transmission assemblies 6, in turn, pulls the two secondary cables 5b, which are physically separate from each other, whose ends are attached to the single fingers 3b, 3c of the prosthetic hand (i.e. the index finger-middle finger and ring finger-little finger pairs). The other end of the primary cable 5a is connected to the mechanism of the opposable finger (thumb) 3a, so that the growing tension in the primary cable 5a causes the thumb to close. The portions of the secondary cables 5b that are engaged with the upper pulleys 65 are connected to the finger pairs 3b, 3c: therefore, an increasing tension in the primary cable 5a will also cause the complete closure of each finger 3a, 3b, 3c into a substantially fist-like configuration, like the one shown in Fig. 11(f).

Describing now the working condition of the prosthetic hand 1 in which the system for stopping the triplet of fingers 3a, 3b, 3c is enabled (i.e. the above-mentioned operative configuration) to allow the finger pair 3c (i.e. ring finger-little finger) to extend while the other fingers 3a, 3b are free to flex, the following should be taken into account.

When the stopping mechanism 100 is activated by the patient by means of a specific EMG signal or another input, it prevents the respective transmission assembly 6 comprising the slider 62 and the pulleys 64, 65, which actuates the finger pair 3c through the respective secondary cable 5b, from being slid downwards along the guide 61 by the primary cable 5a wound by the main motor 4 and by the reducer 4a (see Fig. 10, left side).

This behaviour is made possible by the rotation of the cam 101 controlled by the actuator 103, which moves into the operating condition in which it protrudes from the body 106 and prevents the adjacent transmission assembly 6 from sliding downwards along the guide 61.

In this operating condition, the differential mechanism of the prosthetic hand 1 still allows the other transmission assembly 6 of the finger pair 3b (index finger-middle finger in Fig. 10) of the hand to move downwards along the guide 61, thus permitting the complete opening or closing of the respective fingers 3b.

In this embodiment of the hand 1, only one of the stopping devices 100 (i.e. the left one in Fig.10) locks, when activated, the finger pair 3c (ring finger-little finger) in the fully extended position, thus permitting a "three-finger" precision grasp, wherein only the thumb 3a, the middle finger and the index finger 3b close during the grasping action (Figure 11 (a), 11(c), 11(e)).

Moreover, though not shown in Figure 10, the operation of the prosthetic hand 1 according to the invention envisages that the cam 101, in its operating condition in which it protrudes laterally from the device 100, may prevent the slider 62 of the adjacent transmission assembly 6 from sliding upwards, by acting upon the top side of the slider 62 (with reference to the arrangement shown in Fig. 10).

This locked configuration of the assembly 6 has the effect of keeping the ring finger-little finger pair 3c in the flexed configuration (Fig. 11 (d)). This provides a different version of the three-finger grasp, which is more suited to very accurate tasks, wherein the index and middle fingers 3b in the open (fully extended) position would otherwise interfere.

As can be easily understood, the prosthetic hand 1 according to the invention makes it possible to solve the technical problem at the basis thereof.

In fact, it adds an important functionality to the underactuated hand, i.e. the possibility of differentiated three-finger actuation.

This improves the performance of the hand 1 as regards the ability to grasp and manipulate objects, while still actuating it by means of a single main motor 4.

The invention provides an active, compact and light stopping mechanism, which can be integrated into a poly-articulated, underactuated robotic prosthetic hand using a cable-actuated mechanical-differential mechanism.

The invention allows users to regain one of the most commonly used gripping methods, i.e. the aforementioned three-finger grasp, which until now was only reproducible by fully actuated, but very complex, robotic hands.

This invention, with its mechatronic integration within the poly-articulated, underactuated robotic prosthetic hand, provides inherent differential grip in both enabled and disabled configurations for all the implemented gripping modes. Lastly, the human-machine interface (HMI) is very simple, and the device understands the user's intention and can adjust its own behaviour by controlling the EMG signal.

Thanks to the teaching of the present invention, it is now possible to have an underactuated prosthesis like the one of WO 2019/215577 perform new functions by activating and deactivating the mechanism that drives the cables 5a, 5b during each one of the previously described grasps. Depending on the actions executed for opening and closing the hand 1, the ring finger-little finger pair 3c can remain locked in two different positions: fully extended or fully flexed.

The distinctive technical features of the invention can be summarized as follows.

A very compact, low-power stopping mechanism working in combination with a cable-actuated differential mechanism for an underactuated robotic hand.

A novel method of using a very small motor (the actuator 103 of the stopping devices 100) and a cam-type stopping mechanism 101, combined with a much bigger main grasping motor 4, provides different gripping modes without increased battery consumption or reduced grasping power, which would be the case if two equal, but smaller, motors were employed.

The compactness of the stopping device 100 and of the entire mechanism for stopping the fingers 3a, 3b, 3c permits preserving the anthropomorphic attributes of the hand prosthesis and keeps the total size within 50 % of an average male hand.

Furthermore, the stopping mechanism is light and uses only very simple components; therefore, it provides additional functionalities at a very low cost, especially in relation to the fact that it considerably improves the performance of the hand 1 by adding a further type of finger grasp such as the "pinching" three-finger grasp, with the ring finger and the little finger either fully flexed or fully extended.

At present, the stopping device 100 is only applied to the transmission assembly 6 that supports the pulleys 64 and 65, but it may be applied to any other system of sliders and pulleys in a similar cable-guided differential mechanism.

The present invention permits, being integrated into a tendon-guided differential mechanism, the robotic/prosthetic hand to maintain, in use, its intrinsic differentiability.

All of these features and advantageous effects fall within the scope of the following claims.

## Claims

1. Hand prosthesis (1) comprising a base body (2) that defines a prevalent portion, substantially corresponding to a portion of the back (2a) and/or a portion of the palm (2b) of the hand, a plurality of fingers (3, 3a, 3b, 3c) extending from the base body (2) connected thereto in an articulated manner, and comprising phalanges (31, 32) that are rotatable for finger flexion and extension, a system of sliding cables (5, 5a, 5b) and pulleys (64, 65), associated with the fingers (3, 3a, 3b, 3c) for flexion and extension actuation, **characterized in that** it comprises means (100, 101, 103, 104, 105) for selectively stopping the movements of the fingers (3, 3a, 3b, 3c), so that, when the user commands the actuation of some of them, the other ones will remain in a flexed or extended position, and vice versa.

2. Prosthesis according to claim 1, wherein the stopping means (100, 101, 103, 104, 105), or at least a part (101) thereof, are movable between a first operative condition, in which they stop the movements of the system of cables (5, 5a, 5b) and pulleys (64, 65) associated with one finger (3, 3a, 3b, 3c), and a second non-operative condition, in which they allow such movements.

3. Prosthesis according to claims 1 or 2, wherein the system for actuating the fingers (3, 3a, 3b, 3c) comprises at least one primary cable (5a) connected to a control actuator (4, 4a), a plurality of secondary cables (5b), each one associated with at least one respective finger (3b, 3c), a transmission assembly (6) adapted to allow the primary cable (5a) to control a respective secondary cable (5b), wherein the transmission assembly (6) comprises a slider or another movable element (62), and wherein the stopping means (100, 101, 103, 104, 105) are adapted to prevent the slider or movable element (62) from moving.

4. Prosthesis according to claim 3, wherein the slider or other movable element (62) of the transmission assembly (6) is slidable along a guide (61), and wherein the stopping means (100, 101, 103, 104, 105) comprise a device (100) arranged adjacent to the guide (61).

5. Prosthesis according to any one of the preceding claims, wherein the stopping means (100, 101, 103, 104, 105) comprise a device (100) in which a cam mechanism (101) moves between a non-operative condition, in which the movements of the system of cables (5, 5a, 5b) and pulleys (64, 65) associated with a respective finger (3, 3a, 3b, 3c) are free, and an operative condition, in which the cam (101) cooperates to stop the movements of the system of cables (5, 5a, 5b) and pulleys (64, 65) associated with a respective finger (3, 3a, 3b, 3c).

6. Prosthesis according to claim 5, wherein the stopping device (100) comprises an actuator (103) for actuating the cam (101), which is adapted to hold the cam (101) in said operative condition also when the actuator (103) is deactivated.

7. Prosthesis according to claim 6, wherein the cam mechanism (101) moves between a non-operative condition, in which the cam (101) is substantially aligned with the body (106) of the device (100), and an operative condition, in which the cam (101) protrudes from the body (106) of the device (100) to stop the movements of the system of cables (5, 5a, 5b) and pulleys (64, 65) associated with a respective finger (3, 3a, 3b, 3c) .

8. Prosthesis according to claim 7, wherein the movements of the cam (101) between said operating conditions are associated with those of the actuator (103).

9. Prosthesis according to any one of the preceding claims, comprising at least three fingers (3a, 3b, 3c).

10. Prosthesis according to claim 9, wherein one of the fingers (3a) is opposable to the other two (3b, 3c).

11. Prosthesis according to claims 9 or 10, wherein the fingers that can be selectively actuated comprise index finger-middle finger (3b) and ring finger-little finger (3c) pairs.

## Patentansprüche

1. Handprothese (1), umfassend einen Grundkörper (2), der einen hauptsächlichen Teil definiert, der im Wesentlichen einem Abschnitt des Rückens (2a) und/oder einem Abschnitt der Innenfläche (2b) der Hand entspricht, eine Vielzahl von Fingern (3, 3a, 3b, 3c), die sich von dem Grundkörper (2) weg erstrecken und mit diesem auf gelenkige Weise verbunden sind und Fingerglieder (31, 32) umfassen, die für die Beugung und Streckung der Finger drehbar sind, ein System von Laufseilen (5, 5a, 5b) und Umlenkrollen (64, 65), die den Fingern (3, 3a, 3b, 3c) zur Betätigung der Beugung und Streckung verbunden sind, **dadurch gekennzeichnet, dass** sie Mittel (100, 101, 103, 104, 105) zum selektiven Anhalten der Bewegungen der Finger (3, 3a, 3b, 3c) umfasst, so dass, wenn der Benutzer die Betätigung einiger von ihnen befiehlt, die anderen in einer gebeugten oder gestreckten Position verbleiben und umgekehrt.

2. Prothese nach Anspruch 1, **dadurch gekennzeichnet, dass** die Anhaltemittel (100, 101, 103, 104, 105), oder zumindest ein Teil (101) derselben, zwischen einem ersten operativen Zustand, in dem sie die Bewegungen des Systems aus Seilen (5, 5a, 5b) und Umlenkrollen (64, 65), die einem Finger (3, 3a, 3b, 3c) zugeordnet sind, anhalten, und einem zweiten, nicht-operativen Zustand, in dem sie solche Bewegungen zulassen, beweglich sind.

3. Prothese nach Anspruch 1 oder 2, wobei das System zur Betätigung der Finger (3, 3a, 3b, 3c) mindestens ein primäres Seil (5a), das mit einem Steueraktuator (4, 4a) verbunden ist, eine Vielzahl von sekundären Seilen (5b), von denen jedes mindestens einem jeweiligen Finger (3b, 3c) zugeordnet ist, und eine Übertragungsanordnung (6) umfasst, die dazu geeignet ist, dem primären Seil (5a) zu ermöglichen, ein jeweiliges sekundäres Seil (5b) zu steuern, wobei die Übertragungsanordnung (6) ein Gleitstück oder ein anderes bewegliches Element (62) umfasst, und wobei die Anhaltemittel (100, 101, 103, 104, 105) dazu geeignet sind, die Bewegung des Gleitstücks oder des beweglichen Elements (62) zu verhindern.

4. Prothese nach Anspruch 3, wobei das Gleitstück oder das andere bewegliche Element (62) der Übertragungsanordnung (6) entlang einer Führung (61) verschiebbar ist, und wobei die Anhaltemittel (100, 101, 103, 104, 105) eine Vorrichtung (100) umfassen, die benachbart zu der Führung (61) angeordnet ist.

5. Prothese nach einem der vorhergehenden Ansprüche, wobei die Anhaltemittel (100, 101, 103, 104, 105) eine Vorrichtung (100) umfassen, in welcher sich ein Nockenmechanismus (101) zwischen einem nicht-operativen Zustand, in dem die Bewegungen des Systems von Seilen (5, 5a, 5b) und Umlenkrollen (64, 65), die einem jeweiligen Finger (3, 3a, 3b, 3c) zugeordnet sind, frei sind, und einem operativen Zustand bewegt, in dem der Nocken (101) zusammenwirkt, um die Bewegungen des Systems von Seilen (5, 5a, 5b) und Umlenkrollen (64, 65), die einem jeweiligen Finger (3, 3a, 3b, 3c) zugeordnet sind, anzuhalten.

6. Prothese nach Anspruch 5, wobei die Anhaltevorrichtung (100) einen Aktuator (103) zur Betätigung des Nockens (101) umfasst, der dazu geeignet ist, den Nocken (101) auch dann in dem operativen Zustand zu halten, wenn der Aktuator (103) deaktiviert ist.

7. Prothese nach Anspruch 6, wobei sich der Nockenmechanismus (101) zwischen einem nicht-operativen Zustand, in welchem der Nocken (101) im Wesentlichen mit dem Körper (106) der Vorrichtung (100) ausgerichtet ist, und einem operativen Zustand bewegt, in welchem der Nocken (101) aus dem Körper (106) der Vorrichtung (100) herausragt, um die Bewegungen des Systems von Seilen (5, 5a, 5b) und Umlenkrollen (64, 65) anzuhalten, die einem jeweiligen Finger (3, 3a, 3b, 3c) zugeordnet sind.

8. Prothese nach Anspruch 7, wobei die Bewegungen des Nockens (101) zwischen den operativen Zuständen jenen des Aktuators (103) zugeordnet sind.

9. Prothese nach einem der vorhergehenden Ansprüche, die zumindest drei Finger (3a, 3b, 3c) umfasst.

10. Prothese nach Anspruch 9, wobei einer der Finger (3a) den anderen beiden (3b, 3c) gegenübergestellt werden kann.

11. Prothese nach Anspruch 9 oder 10, wobei die Finger, die selektiv betätigt werden können, die Paare Zeigefinger-Mittelfinger (3b) und Ringfinger-Kleinfinger (3c) umfassen.

## Revendications

1. Prothèse de main (1) comprenant un corps de base (2) définissant une partie prédominante correspondant sensiblement à une partie du dos (2a) et/ou à une partie de la paume (2b) de la main, une pluralité de doigts (3, 3a, 3b, 3c) s'étendant depuis le corps de base (2) et reliés à celui-ci de manière articulée, et comprenant des phalanges (31, 32) rotatives pour la flexion et l'extension des doigts, un système de câbles coulissants (5, 5a, 5b) et de poulies (64, 65), associés aux doigts (3, 3a, 3b, 3c) pour l'actionnement en flexion et en extension, **caractérisée en ce qu'**elle comprend des moyens (100, 101, 103, 104, 105) pour arrêter sélectivement les mouvements des doigts (3, 3a, 3b, 3c), de sorte que, lorsque l'utilisateur commande l'actionnement de certains d'entre eux, les autres d'entre eux resteront en position fléchie ou étendue, et vice versa.

2. Prothèse selon la revendication 1, dans laquelle les moyens d'arrêt (100, 101, 103, 104, 105), ou au moins une partie (101) de ceux-ci, sont mobiles entre un premier état fonctionnel, dans lequel ils arrêtent les mouvements du système de câbles (5, 5a, 5b) et de poulies (64, 65) associés à un doigt (3, 3a, 3b, 3c), et un second état non fonctionnel, dans lequel ils autorisent de tels mouvements.

3. Prothèse selon la revendication 1 ou 2, dans laquelle le système d'actionnement des doigts (3, 3a, 3b, 3c) comprend au moins un câble primaire (5a) relié à un actionneur de commande (4, 4a), une pluralité de câbles secondaires (5b), chacun associé à au moins un doigt respectif (3b, 3c), un ensemble de transmission (6) adapté pour permettre au câble primaire (5a) de commander un câble secondaire respectif (5b), dans laquelle l'ensemble de transmission (6) comprend un curseur ou un autre élément mobile (62), et dans laquelle les moyens d'arrêt (100, 101, 103, 104, 105) sont adaptés pour empêcher le curseur ou l'élément mobile (62) de se déplacer.

4. Prothèse selon la revendication 3, dans laquelle le curseur ou autre élément mobile (62) de l'ensemble de transmission (6) peut coulisser le long d'un guide (61), et dans laquelle les moyens d'arrêt (100, 101, 103, 104, 105) comprennent un dispositif (100) disposé adjacent au guide (61).

5. Prothèse selon l'une quelconque des revendications précédentes, dans laquelle les moyens d'arrêt (100, 101, 103, 104, 105) comprennent un dispositif (100) dans lequel un mécanisme à came (101) se déplace entre un état non opérationnel, dans lequel les mouvements du système de câbles (5, 5a, 5b) et de poulies (64, 65) associés à un doigt respectif (3, 3a, 3b, 3c) sont libres, et un état opérationnel, dans lequel la came (101) coopère pour arrêter les mouvements du système de câbles (5, 5a, 5b) et de poulies (64, 65) associés à un doigt respectif (3, 3a, 3b, 3c).

6. Prothèse selon la revendication 5, dans laquelle le dispositif d'arrêt (100) comprend un actionneur (103) pour actionner la came (101), qui est adapté pour maintenir la came (101) dans ledit état opérationnel également lorsque l'actionneur (103) est désactivé.

7. Prothèse selon la revendication 6, dans laquelle le mécanisme à came (101) se déplace entre un état non opérationnel, dans lequel la came (101) est sensiblement alignée avec le corps (106) du dispositif (100), et un état opérationnel, dans lequel la came (101) fait saillie du corps (106) du dispositif (100) pour arrêter les mouvements du système de câbles (5, 5a, 5b) et de poulies (64, 65) associés à un doigt respectif (3, 3a, 3b, 3c).

8. Prothèse selon la revendication 7, dans laquelle les mouvements de la came (101) entre lesdits états d'opération sont associés à ceux de l'actionneur (103).

9. Prothèse selon l'une quelconque des revendications précédentes, comprenant au moins trois doigts (3a, 3b, 3c).

10. Prothèse selon la revendication 9, dans laquelle l'un des doigts (3a) est opposable aux deux autres (3b, 3c).

11. Prothèse selon les revendications 9 ou 10, dans laquelle les doigts pouvant être actionnés sélectivement comprennent des paires index-majeur (3b) et annulaire-auriculaire (3c).
